# EUROPEAN PATENT APPLICATION

(11) **EP 2 119 786 A1**
(43) Date of publication of application: **18.11.2009**
(21) Application number: 08156102.9
(22) Date of filing: 13.05.2008
(51) Int. Cl.: C12N 15/82, A01H 5/00, C12N 15/54

(54) **Increased production of health-promoting compounds in plants**

(71) Applicant: Expressive Research B.V., 6709 PA Wageningen (NL)
(72) Inventor: de Boer, Anne Douwe, 6621 AC Dreumel (NL); Passarinho, Paul Alexandre, 6624 BC Heerewaarden (NL)
(74) Representative: Hatzmann, Martin

(57) **Abstract**

The present invention relates to a method to enhance the health-promoting capacity of a plant by increasing expression of a regulatory gene product, preferably wherein said regulatory gene product is a receptor kinase, more preferably a RKS receptor or a mutein thereof or a chimeric RKS receptor. Such a method comprises providing a plant with a nucleotide sequence encoding said RKS receptor, but alternatively also a sequence encoding a truncated RKS protein can be used. Said RKS gene can be chosen from the group consisting of RKS subgroup I (RKS1, RKS4, RKS5, RKS7, RKS11, and RKS14), more specifically RKS4 and truncated RKS4 and chimeric receptors comprising the extracellular domain of RKS4. A further preferred embodiment is a method according to the invention wherein enhancing the health-promoting capacity of a plant comprises production in said plant of health-promoting substances selected from the group of polyphenols, glucosinolates, isoprenoids, and GABA and analogs, more preferably chosen from the group consisting of quercetin, kaempferol, sinapic acid, gallic acid, GABA and sinigrin.

In another embodiment the invention is related to a method to produce a health-promoting substance in a plant by introducing into said plant a gene coding for a regulatory gene product, preferable a receptor kinase, more preferably an RKS gene. Further part of the invention is the use of an RKS gene for the production of health-promoting substances in a plant.

Also part of the invention is a plant with an enhanced health-promoting capacity produced by the method according to the invention.

## Description

### FIELD OF THE INVENTION

The invention relates to the field of plant biotechnology, more particularly to the field of production of chemical compounds in plants, especially to the production of health-promoting compounds in plants.

### BACKGROUND

Under its broadest definition, food biotechnology started thousands of years ago when primitive man advanced from hunting and gathering food to farming. For thousands of centuries, plant breeders selected, sowed and harvested seeds to produce enough food to sustain life and to develop desirable traits in their crops such as better taste, richer colour and hardier plants.

At the beginning of the previous century, farmers carefully selected plants with beneficial traits and began breeding them together, creating new varieties and hybrids, new plants with some of the qualities of each of the parents. Though they did not understand the underlying scientific principles involved, early farmers have been harnessing biotechnology for centuries to make or modify plants and food products.

Since the beginning of time, farmers have sought to improve the quality and quantity of food crops through plant selection and hybridisation. By introducing a gene (or genes) through genetic modification, beneficial changes may be made to plant crops. Examples include:
- Consistently high-yielding oil palms
- Potatoes and tomatoes with a higher content of solids, making the plants more suitable for food processing
- Tomatoes, squash and potatoes with higher levels of nutrients such as vitamins A, C and E.
- The bark of cinchona trees the source of a variety of alkaloids, among which quinine, an anti-fever agent especially useful in treating malaria.
- Corn and soya beans containing higher levels of essential amino acids.
- Potatoes with higher levels of essential amino acids.
- Oil seeds with lower levels of saturated fat.
- Garlic cloves with more allicin, an active ingredient that is being researched for a potential role in helping to lower cholesterol.
- Strawberries with increased levels of natural agents that are being studied for their role in helping to fight cancer.
- Slow-ripening tomatoes, peppers and tropical fruits with better keeping qualities and better flavour.
- Crops that can grow in very low temperatures.
- Animal feed crops with improved levels of proteins.

Crop improvements like these can help provide an abundant supply of quality food. Increasing the nutritional content of staple foods could help certain populations get more nutrients without having to change their diets significantly.

Recently, some groups of compounds that are produced in plants, like polyphenols, glucosinolates and isoprenoids, have been attributed health-promoting qualities.
- *Role of polyphenols in human health (phenylpropanoids and tannins (e.g. gallic acid)):* polyphenols mainly prevent age-related diseases such as cancer and cardiovascular diseases (J. Med. Food (2005) 8 (3): 281-290). In addition they are already part of our diet (i.e. vegetables and fruits as well as plant extracts, e.g. tea, coffee or red wine) and have a low toxicity all of which makes them good candidate chemoprotective agents. Anticarcinogenic effects are thought to be achieved through antioxidant activity (i.e. free radical scavenging) and changes in enzymatic activity (Phase II (i.e. detoxification) enzymes) that lead towards inactivation and elimination of mutagens or the regulation of cell proliferation and apoptosis (Curr. Med. Chem. (2001) 8(11): 1245-1255; Tox. In Vitro (2006) 20(2): 187-210). The polyphenols are also known to prevent cardiovascular diseases, through for example antiatherogenic effects (e.g. kaempferol - J. Agric. Food Chem. (2007) 55(24): 9969-9976) or hypotensive properties (e.g. kaempferol - Phytotherapy Res. (1998) 11(8): 564-567). Also a hypoglycaemic effect has been described (e.g. for kaempferol - J. Nat. Prod. (2004) 67(5): 829-832). Further, anti-inflammatory, anti-allergic effects (e.g. quercetin - Allergol. Int. (2007) 56(2): 113-123) and neuroprotection (Brain Res. (2007) 1173: 117-125; Braz. J. Med. Biol. Res. (2003) 36(12): 1613-1620) are reported.
- *Role of glucosinolates in human health:* glucosinates may protect against human cancers, especially those of the gastrointestinal tract and lung. Their anticarcinogenic activity is achieved through the induction of Phase II enzymes and the inhibition of Phase I enzymes (responsible for the activation of cancer development) and, like the above discussed polyphenols, through the regulation of cell proliferation and apoptosis (Phytochem Rev. (2007) Online First™ publication DOI 10.1007/s11101-007-9072-2). Aliphatic glucosinolates such as sinigrin are likely to either induce Phase II enzymes or to inhibit Phase I enzymes (J. Sci. Food Agric. (2006) 86(4): 528-538) as well as to inhibit metastasis and tumour formation (Exp. Biol. Med. (Maywood) (2006) 231(4):421-430).
- *Role of isoprenoids in human health (such as carotenoids (e.g. vitamin A), phytosterols, vitamin E, terpenoid):* Isoprenoids are probably the most diverse and largest family of natural products and include many important drugs (e.g. taxol (J. Am. Chem. Soc. (1971) 93(9): 2325-2327), artemisinin (Mini Rev. Med. Chem. (2007) 7(4): 411-422)), valuable flavor and fragrance compounds (e.g. menthol), as well as pigments, antioxidants and steroids. Many of those can be used for the prevention of cardiovascular diseases and cancer: isoprenoids are useful cancer chemopreventive agents and suppress tumour growth by inhibiting HMG-CoA reductase (Curr. Cancer Drug Targets (2006) 6(1): 15-37). A known effect of phytosterols is their hypocholesterolemic activity (Am. J. Clin. Nutr. (2003) 77(6): 1385-1589).

Modern biotechnology enables to create plants that overproduce one or more specific compounds of the above groups, There is however need for alternative strategies for producing plants which overproduce health-promoting compounds.

### SUMMARY OF THE INVENTION

The present invention now relates to a method to enhance the health-promoting capacity of a plant by increasing expression of a regulatory gene product, preferably wherein said regulatory gene product is a receptor kinase, more preferably a RKS receptor or a mutein thereof or a chimeric RKS receptor. Such a method comprises providing a plant with a nucleotide sequence encoding said RKS receptor, but alternatively also a sequence encoding a truncated RKS protein can be used. Said RKS gene can be chosen from the group consisting of RKS subgroup I (RKS1, RKS4, RKS5, RKS7, RKS11, and RKS14), more specifically RKS4 and truncated RKS4 and chimeric receptors comprising the extracellular domain of RKS4. A further preferred embodiment is a method according to the invention wherein enhancing the health-promoting capacity of a plant comprises production in said plant of health-promoting substances selected from the group of polyphenols, glucosinolates, isoprenoids, and GABA and analogs, more preferably chosen from the group consisting of quercetin, kaempferol, sinapic acid, gallic acid, GABA and sinigrin.

In another embodiment the invention is related to a method to produce a health-promoting substance in a plant by introducing into said plant a gene coding for a regulatory gene product, preferable a receptor kinase, more preferably an RKS gene. Further part of the invention is the use of an RKS gene for the production of health-promoting substances in a plant.

Also part of the invention is a plant with an enhanced health-promoting capacity produced by the method according to the invention.

### LEGENDS TO THE FIGURES

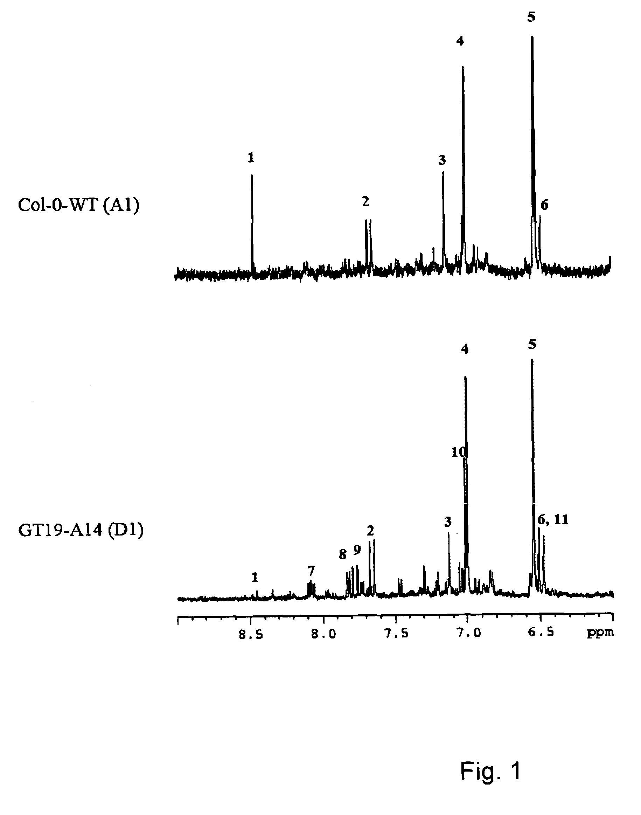

**Figure 1****. Example of 1H-NMR spectra in the range of** δ **6.0 -** δ **9.0.** Comparison between the wild-type Col-0 and the transgenic line GT19-A14, samples A1 and D1, respectively. **1;** formic acid, **2;** H-7 of sinapic acid analogues, **3;** gallic acid, **4;** H-2 and H-6 of sinapic acid analogue 1, **5,** fumaric acid, **6;** H-8 of sinapic acid analogue 1, **7;** H-2' and H-6' of kaempferol-3-*O-*glucose-7-*O*-rhamnoside, **8;** 7; H-2' and H-6' of kaempferol-3,7-*O*-dirhamnoside, **9;** H-7 of sinapic acid analogue 2, **10;** H-2 and H-6 of sinapic acid analogue 2 **11;** H-8 of sinapic acid analogue 2.
**Figure 2****. Schematic view of the GABA shunt metabolic pathway** (adapted from TIPS (2004) 9(3): 110-115). Boxed compounds are more abundant in RKS4 transgenic plants, whereas shaded compounds are less abundant as compared to the wild-type.
**Figure 3****. Metabolic pathways influenced by modulation of the *RKS4* gene in Arabidopsis transgenic plants.**

Transcriptional changes in RKS4 transgenic plants after Pseudomonas infection were analysed with the 'Pathway Tools Omics Viewer' at The Arabidopsis Information Resource (www.arabidopsis.org). Gene names (AGI number) are indicated for each of the depicted reactions and a colour code is applied for reactions in which the differential genes are involved. The colour corresponds to the expression level that is also indicated next to the AGI number that is shaded in grey. The values indicated are the fold-expression changes between the transgenic line and the wild-type 48 hours post infection.

Individual pathways are framed in rounded boxes and their name is indicated in bold. A grey background is added to pathways which are interconnected in a super pathway: Isoprenoid synthesis in **A** and Phenylpropanoid synthesis in **B**. Intermediate or derived pathways are boxed and if shaded linked to other pathways in which differentially regulated genes were found.

### DETAILED DESCRIPTION

The present invention surprisingly shows that it is possible to increase the production and subsequently the content of various health-promoting substances in plants. As used herein 'health-promoting substances' are chemical compounds that naturally occur in plants and to which a therapeutic effect is attributed. Some of these health-promoting substances with their therapeutic effects are listed below:

**Kaempferol** is a strong antioxidant and helps to prevent oxidative damage of cells, lipids and DNA. Kaempferol seems to prevent arteriosclerosis by inhibiting the oxidation of low density lipoproteins and the formation of platelets in the blood. Studies have also confirmed that kaempferol acts as a chemopreventive agent. For example kaempferol inhibits monocyte chemoattractant protein (MCP-1). MCP-1 plays a role in the initial steps of atherosclerotic plaque formation (Pharmacol. Rep. (2005) 57(1): 107-112). Kaempferol also seems to act synergistically with quercetin in reducing cell proliferation of cancer cells, meaning that the combined treatments with quercetin and kaempferol are more effective than the additive effects of each flavonoid (In Vivo (2005) 19(1): 69-76).

**Quercetin** has many health promoting effects, including improvement of cardiovascular health and reduction of risk for cancer, mostly through its strong antioxidant action that helps to combat cell damaging free radicals.

Quercetin has anti-inflammatory and anti-allergic effects through the inhibition of enzymes, such as lipoxygenase, and the inhibition of inflammatory mediators. Quercetin also inhibits the release of histamine, which causes congestion, by basophiles and mast cells (Allergol. Int. (2007) 56(2): 113-123). As many other flavonoids, quercetin prevents the oxidation of LDL (bad) cholesterol (J. Nutrition. (2000) 130(9): 2243-2250). Studies have shown that quercetin reduces the cancer risk of prostate, ovary, breast, gastric and colon cells (J. Nutrition (2006) 136(5): 1178-1184; Nutr. J. (2005) 4(l):11). Quercetin, as kaempferol, also seems to reduce the production of uric acid, by inhibiting the xanthine oxidase, thereby easing gout symptoms (Biosci. Biotechnol. Biochem. (1999) 63(10): 1787-1790). Studies have shown an improved lung function and lower risk of certain respiratory diseases (asthma and bronchitis - Inflamm. Res. (2007) 56(10):402-408).

**Sinapic acid** has free-radical-scavenging activity (Eur. J. Lipid Sci. Technol. (2006) 108(3): 239-248) and protection against cerebral neuronal damage and death (Biol. Pharm. Bull. (2007) 30(3):514-519).
Used in the production of cosmetic and/or dermatological preparations for protecting skin against aging, improving wound healing, treating sensitive skin and alleviating neurogenic inflammation (Patent no. EP1437117). Its anxiolytic activity through the potentiation of the GABA response makes it an interesting alternative to the currently used benzodiazepines that have many side effects and are costly (Life Sci. (2007) 81(3):234-240).

**Gallic acid** seems to have anti-microbial (Ethnopharmacol. (2002) 79(2):165-168) and anti-viral properties (Antiviral Res. (2007) 73(2):85-91). Gallic acid acts as an antioxidant and helps to protect our cells against oxidative damage. Gallic acid was found to show cytotoxicity against cancer cells, without harming healthy cells (Int. J. Oncol. (2007) 30(3):605-613). It also has anti-allergic properties (Toxicol. Sci. (2006) 91(1):123-131).

**Gamma-AminoButyric Acid** (GABA) is an inhibitory neurotransmitter which binding to the GABA_{A} receptor is at the basis of the effect of anxiolytics such as benzodiazepines that increase this process. Benzodiazepines also have an anticonvulsant activity that again is mediated by GABA. Consequently GABA is used for treatment of e.g. depressions, epileptic seizures, but also hypertension. In addition GABA is also involved in stimulating the production of (human) growth hormone (Neuroendocrinology (2002) 76(3):170-177) and as such has been shown to being able to slow down aging, increase fat loss and muscle buildup
(http://thesupplementguide.org/g/Emotional_Health_Cognition/gabagammaaminobutyric-acid-12.html). It may have an additive effect in combination with sinapic acid (Life Sci. (2007) 81(3):234-240).

**Sinigrin** is an aliphatic glucosinolate with anticarcinogenic activity (Phytochem Rev. (2007) Online First™ publication DOI 10.1007/s11101-007-9072-2), likely to either induce Phase II enzymes or to inhibit Phase I enzymes (J. Sci. Food Agric. (2006) 86(4): 528-538) as well as to inhibit metastasis and tumour formation (Exp. Biol. Med. (Maywood) (2006) 231(4):421-430).

The production of the above and other compounds is effected by modulation of the expression of regulatory gene products. "Regulatory gene products" as used herein are defined as key elements of signal transduction cascades leading to the modulation of cellular processes such as gene activation or metabolic changes as a result of one or more external or internal signals (e.g. an environmental stimulus such as an invading pathogen or temperature). In particular with regulatory gene products receptor-like kinases, more specifically RKS receptors, including RKS receptors of subgroup I and more specifically the RKS4 receptor are meant.

The RKS family (Receptor Kinase like SERK) forms the LRRII RLK subfamily as defined by (PNAS (2001) 98:10763-10768) based on the copy number and structural arrangement of the Leucine-Rich-Repeats (LRRs). It consists of 14 members in Arabidopsis for which the corresponding genes were first described (see WO 01/29240 and WO 2004/007712) and are listed below.

| *Gene name* | *AGI code* |
|---|---|
| *RKS0* | *At1g71830* |
| *RKS1* | *At1g60800* |
| *RKS2* | *At5g65240* |
| *RKS3* | *At5g63710* |
| *RKS4* | *At2g23950* |
| *RKS5* | *At5g45780* |
| *RKS6* | *At5g10290* |
| *RKS7* | *At5g16000* |
| *RKS8* | *At1g34210* |
| *RIS10* | *At4g33430* |
| *RKS11* | *At4g30520* |
| *RKS12* | *At2g13800* |
| *RKS13* | *At2g13790* |
| *RKS14* | *At3g25560* |

The RKS receptors all contain the 3 characteristic domains of this subfamily: an extracellular domain consisting of 5 LRRs arranged in tandem in a single continuous block, a transmembrane domain and an intracellular kinase domain. The first four LRRs of the extracellular domain are full-length (24 amino acids) whereas LRR5 is truncated and consists of 16 residues only and in RKS3 LRR4 has been deleted. Intron position and number is conserved except in the extracellular domain of RKS3 and in the kinase domain of RKS2 and RKS6.

Three subgroups can be defined based on kinase domain sequence (Zhang et al., 2006, J. Mol. Evol. 63: 612-621; WO 01/29240 and WO 2004/007712). Subgroup I consists of RKS1, RKS4 RKS5, RKS7, RKS11 and RKS14, subgroup II of: RKS0, RKS8, RKS12 and RKS13 and subgroup III of RKS2, RKS3 and RKS6. Furthermore subgroup II has a common SPP box preceding the transmembrane domain (Schmidt et al. (1997) Dev. 124: 2049-2062) absent from the other subgroups. On the other hand subgroup I distinguishes itself from the others by for example the presence of the 'PSQ' motif in LRR1 or the 'LQNNxI' motif in LRR2, that are conserved across species.

Orthologous receptors from other plants and the coding sequences for these receptors, which have not yet been isolated, can be used as well. It is believed that these coding sequences will be homologous to the sequences disclosed in the above mentioned references. Thus, in principle any nucleotide sequence, which is homologous to said sequences and which codes for a protein that at least functions as an RKS receptor would be useful. These nucleotide sequences can be isolated from plants expressing orthologous receptors, however, these nucleotide sequences can also be made by modifying the existing nucleotide sequences, which then would code for muteins of the already known receptors.

"Muteins" of the receptors of the invention are proteins that are obtained from the already known receptors by replacing, adding and/or deleting one or more amino acids, while still retaining their function as receptor for systemic signalling compounds. Such muteins can readily be made by protein engineering, e.g. by changing the open reading frame capable of encoding the protein so that the amino acid sequence is thereby affected. As long as the changes in the amino acid sequences do not altogether abolish the activity of the protein such muteins are embraced in the present invention. Further, it should be understood that muteins should be derivable from the known receptors while retaining biological activity, i.e. all, or a great part of the intermediates between the mutein and the amino acid sequence of the protein depicted in the above cited references should be capable of being induced by systemic signalling compounds. A great part would mean 30% or more of the intermediates, preferably 40% of more, more preferably 50% or more, more preferably 60% or more, more preferably 70% or more, more preferably 80% or more, more preferably 90% or more, more preferably 95% or more, more preferably 99% or more.

Thus, also part of the invention are receptors which are at least 70% identical to known RKS proteins, but more preferably more than 80% identical, more preferably more than 90% identical and most preferably more than 95% identical to the above discussed known receptors. For calculation of percentage identity the BLAST algorithm can be used (Nucl. Acids Res., 1997, 25, 3389-3402) using default parameters or, alternatively, the GAP algorithm (J. Mol. Biol., 1970, 48, 443-453), using default parameters, which both are included in the Wisconsin Genetics Software Package, Genetics Computer Group (GCG), 575 Science, Madison, Wisconsin, USA. BLAST searches assume that proteins can be modelled as random sequences. However, many real proteins comprise regions of non-random sequences, which may be homopolymeric tracts, short-period repeats, or regions enriched in one or more amino acids. Such low-complexity regions may be aligned between unrelated proteins even though other regions of the protein are entirely dissimilar. A number of low-complexity filter programs can be employed to reduce such low-complexity alignments. For example, the SEG (Comput. Chem., 1993, 17, 149-163) and XNU (Comput. Chem., 1993, 17, 191-201) low-complexity filters can be employed alone or in combination.

As used herein, 'sequence identity' or 'identity' or 'homology' in the context of two protein sequences (or nucleotide sequences) includes reference to the residues in the two sequences which are the same when aligned for maximum correspondence over a specified comparison window. When percentage of sequence identity is used in reference to proteins it is recognised that residue positions which are not identical often differ by conservative amino acid substitutions, where amino acids are substituted for other amino acid residues with similar chemical properties (e.g. charge or hydrophobicity) and therefore do not change the functional properties of the molecule. Where sequences differ in conservative substitutions, the percentage sequence identity may be adjusted upwards to correct for the conservative nature of the substitutions. Sequences, which differ by such conservative substitutions are said to have 'sequence similarity' or 'similarity'. Means for making these adjustments are well known to persons skilled in the art. Typically this involves scoring a conservative substitution as a partial rather than a full mismatch, thereby increasing the percentage sequence identity. Thus, for example, where an identical amino acid is given a score of 1 and a non-conservative substitution is give a score of zero, a conservative substitution is given a score between 0 and 1. The scoring of conservative substitutions is calculated, e.g. according to the algorithm of Meyers and Miller (Computer Applic. Biol. Sci., 1998, 4, 11-17).

As used herein, 'percentage of sequence identity' means the value determined by comparing two optimally aligned sequences over a comparison window, wherein the portion of the amino acid sequence or nucleotide sequence in the comparison window may comprise additions or deletions (i.e., gaps) as compared to the reference sequence for optimal alignment of the two sequences. The percentage is calculated by determining the number of positions at which the identical amino acid or nucleic acid base residue occurs in both sequences to yield the number of matched positions, dividing the number of matched positions by the total number of positions in the window of comparison and multiplying the result by 100 to yield the percentage of sequence identity.

In general not all amino acids of a protein and not all nucleotides of a nucleotide sequence are equally well interchangeable. In most case proteins have one or more regions which are important or crucial for the function. For the RKS receptors of the invention it is easy to determine the less variable regions by aligning the sequences (which can be found in WO 04/007712) and determining so-called consensus sequences, i.e. parts of the protein which are well conserved between homologous sequences with the same function. When trying to design variants (or muteins) of the RKS receptors, these consensus sequences should preferably be kept intact, while other regions may be varied more. In the group of RKS receptors the most preferred are RKS1, RKS4, RKS5, RKS7, RKS11 and RKS14. This subgroup I shares specific consensus sequences described above.

Very important is to mention that partial receptors, e.g. only (parts of the) extracellular domain or only the intracellular domain or fragments thereof of the receptor like kinases are able to effect a constitutive active receptor function. Earlier obtained results indicate that the N-terminal part of RKS4 acts as a constitutive activator of downstream cascades. When, in the present invention the N-terminal part of an RKS receptor is mentioned, the extracellular domain of said RKS receptor is meant. A person skilled in the art will understand what part of the receptor is meant by the extracellular domain. Besides, in WO 04/007712 the extracellular domains of the RKS receptors have been indicated.

A further embodiment of the invention is formed by chimeric receptors, in which the ligand binding part of the above mentioned receptors is replaced by a ligand binding part of another receptor. In this way it is possible to induce the production of downstream compounds, among which the indicated health-promoting substances, by application of an inducer that is able to trigger the ligand binding part of the receptor.

The nucleotide sequences will need to be expressed in the plant(s) into which they are transformed. For this a genetic construct (expression cassette) that comprises an expressible nucleotide sequence is needed. The expression of the nucleotide sequence depends on the operational elements contained in such a construct, such as a promoter, a terminator, and enhancing elements.

The term "promoter" is intended to mean a short DNA sequence to which RNA polymerase and/or other transcription initiation factors bind prior to transcription of the DNA to which the promoter is functionally connected, allowing transcription to take place. The promoter is usually situated upstream (5') of the coding sequence. In its broader scope, the term "promoter" includes the RNA polymerase binding site as well as regulatory sequence elements located within several hundreds of base pairs, occasionally even further away, from the transcription start site. Such regulatory sequences are, e.g. sequences that are involved in the binding of protein factors that control the effectiveness of transcription initiation in response to physiological conditions. The promoter region should be functional in the host cell and preferably corresponds to the natural promoter region of the receptor protein. However, any heterologous promoter region can be used as long as it is functional in the host cell where expression is desired. The heterologous promoter can be either constitutive, tissue or developmental specific or regulatable. A constitutive promoter such as the CaMV 35S promoter or T-DNA promoters, all well known to the person skilled in the art, are promoters, which are subjected to substantially no regulation such as induction or repression, but which allow for a steady and substantially unchanged transcription of the DNA sequence to which it is functionally bound in all or most of the active cells of the organism provided that other requirements for the transcription to take place are fulfilled. A tissue-specific promoter is a promoter, which restricts the expression of the coding sequence to a limited part of the plant, i.e. a special tissue and/or a special cell type. An often used tissue-specific promoter is the Rubisco promoter (which is specific for green parts of the plants). A regulatable or inducible promoter is a promoter of which the function is regulated by one or more factors, either internally present or externally added (Trends in biotechnology 2005, 23, 283-290). In the absence of an inducer, the DNA sequence will either not be transcribed or will be transcribed at a reduced level relative to transcription levels in the presence of an inducer. In certain instances, a factor may bind specifically to an inducible promoter to activate transcription, said factor being present in an inactive form and convertible (either directly or indirectly) to an active form by the inducer. The inducer may be a chemical/biochemical agent, such as a protein, metabolite (sugar, alcohol, etc.) a growth regulator, a herbicide, or a phenolic compound. Alternatively, the inducer may be a directly imposed physiological stress (for example, heat, salt, wounding, toxic elements, etc.) or an indirectly imposed physiological stress (for example, the action of a pathogen or disease agent, such as a virus). A plant cell containing an inducible promoter may be exposed to an inducer by external application of the inducer to the cell such as by spraying, watering, heating, or similar methods. Examples of inducible promoters include the inducible 70 kD heat shock promoter of Drosophila melanogaster (Ann. Rev. Genet., 1985, 19, 297-323) and the alcohol dehydrogenase promoter which is induced by ethanol (Nagao, R.T. et al., in: Miflin, B.J. (ed.) Oxford Surveys of Plant Molecular and Cell Biology, Vol. 3., pp. 384-438, Oxford Univ. Press, 1986). Examples of promoters that are inducible by a simple chemical are described in Gurr and Rushton (Trends in biotechnology 2005, 23, 283-290), WO 90/08826, WO 93/21334, WO 93/031294 and WO 96/37609.

A terminator is a short piece of DNA that serves to terminate the transcription of the DNA into RNA and is preferably selected from the group consisting of plant transcription terminator sequences, bacterial transcription terminator sequences and plant virus terminator sequences known to those skilled in the art.

Enhancing elements (such as the 35S enhancer) and other elements like scaffold attachment regions (SARs) can be used to increase expression of the genes of the invention. It is also possible to boost expression by introducing an intron (e.g. the Adh-intron) in the open reading frame or to use viral enhancer sequences.

The term "gene" is used to indicate a DNA sequence, which is involved in producing a polypeptide chain and which includes regions preceding and following the coding region (5'-upstream and 3'-downstream sequences) as well as intervening sequences, the so-called introns, which are placed between individual coding segments (so-called exons) or in the 5'-upstream or 3'-downstream region. The 5'-upstream region may comprise a regulatory sequence that controls the expression of the gene, typically a promoter. The 3'-downstream region may comprise sequences, which are involved in termination of transcription of the gene and optionally sequences responsible for polyadenylation of the transcript and the 3' untranslated region.

In eukaryotic cells, an expression cassette usually further comprises a transcriptional termination region located downstream of the open reading frame, allowing transcription to terminate and polyadenylation of the primary transcript to occur. In addition, the codon usage may be adapted to accepted codon usage of the host of choice. The principles governing the expression of a DNA construct in a chosen host cell are commonly understood by those of ordinary skill in the art and the construction of expressible DNA constructs is now routine for any sort of host cell, be it prokaryotic or eukaryotic.

In order for the open reading frame to be maintained in a host cell it will usually be provided in the form of a replicon comprising said open reading frame according to the invention linked to DNA, which is recognised and replicated by the chosen host cell. Accordingly, the selection of the replicon is determined largely by the host cell of choice. Such principles as govern the selection of suitable replicons for a particular chosen host are well within the realm of the ordinary skilled person in the art.

A special type of replicon is one capable of transferring itself, or a part thereof, to another host cell, such as a plant cell, thereby co-transferring the open reading frame according to the invention to said plant cell. Replicons with such capability are herein referred to as vectors. An example of such vector is a Ti-plasmid vector, which, when present in a suitable host, such as *Agrobacterium tumefaciens,* is capable of transferring part of itself, the so-called T-region, to a plant cell. Different types of Ti-plasmid vectors (vide: EP 0 116 718 B1) are now routinely being used to transfer DNA sequences into plant cells, or protoplasts, from which new plants may be generated which stably incorporate said DNA in their genomes. A particularly preferred form of Ti-plasmid vectors are the so-called binary vectors (as claimed in EP 0 120 516 B1 and US 4,940,838) such as pGreen (Hellens, R.P. et al., 2000, Plant mol. Biol. 42:819-832). Other suitable vectors, which may be used to introduce DNA according to the invention into a plant host, may be selected from the viral vectors, e.g. non-integrative plant viral vectors, such as derivable from the double stranded plant viruses (e.g. CaMV) and single stranded viruses, Gemini viruses and the like. The use of such vectors may be advantageous, particularly when it is difficult to stably transform the plant host. Such may be the case with woody species, especially trees and vines.

The expression "host cells incorporating a DNA sequence according to the invention in their genome" shall mean to comprise cells, as well as multicellular organisms comprising such cells, or essentially consisting of such cells, which stably incorporate said DNA into their genome thereby maintaining the DNA, and preferably transmitting a copy of such DNA to progeny cells, be it through mitosis or meiosis. According to a preferred embodiment of the invention plants are provided, which essentially consist of cells that incorporate one or more copies of said DNA into their genome, and which are capable of transmitting a copy or copies to their progeny, preferably in a Mendelian fashion. By virtue of the transcription and translation of the DNA according to the invention in some or all of the plant's cells, those cells that are capable of producing regulatory gene products such RKS receptors will show an enhanced production of health-promoting substances.

Transformation of plant species is now routine for an impressive number of plant species, including both the Dicotyledonous as well as the Monocotyledonous. In principle any transformation method may be used to introduce chimeric DNA according to the invention into a suitable ancestor cell, as long as the cells are capable of being regenerated into whole plants. Methods may suitably be selected from the calcium/polyethylene glycol method for protoplasts (, Nature, 1982, 296, 72-74; Plant Mol. Biol., 1987, 8, 363-373), electroporation of protoplasts (Bio/Technol., 1985, 3, 1099-1102), microinjection into plant material ( Mol. Gen. Genet., 1986, 202, 179-185), (DNA or RNA-coated) particle bombardment of various plant material ( Nature, 1987, 327, 70), infection with (non-integrative) viruses and the like. A preferred method according to the invention comprises Agrobacterium-mediated DNA transfer. Especially preferred is the use of the so-called binary vector technology as disclosed in EP A 120 516 and U.S. Patent 4,940,838.

Transformation can be facilitated by the use of selectable or screenable markers to discriminate between transformed plants or plant cells and non-transformed plants or plant cells. However, possibly so-called marker-free transformation protocols, such as for instance described in WO 01/29240, can be used.

Generally, after transformation plant cells or cell groupings are selected for the presence of one or more markers which are encoded by plant expressible genes co-transferred with the nucleic acid sequence according to the invention, where after the transformed material is regenerated into a whole plant. Genes which can be used as marker genes can be roughly divided in antibiotic resistance marker genes, such as nptII (giving resistance to kanamycin) and hpt (giving resistance to phosphonotricin), and developmental or metabolic selection marker genes, such as the trehalase gene, the mannose gene (both metabolic markers) and the IPT gene. For marker-free transformation it is possible to use the previously described T/R system based on transient activity of regenerating gene products (W09743427) or marker excision systems (such as, e.g. described in WO 02/097102), or stable integration of inducible regenerating gene products. A new approach to obtain marker-free plants has been described in WO 03/010319.

Although considered somewhat more recalcitrant towards genetic transformation, monocotyledonous plants are amenable to transformation and fertile transgenic plants can be regenerated from transformed cells or embryos, or other plant material. Presently, preferred methods for transformation of monocots are microprojectile bombardment of embryos, explants or suspension cells, and direct DNA uptake or electroporation (Shimamoto, et al, 1989, Nature 338, 274-276). Transgenic maize plants have been obtained by introducing the *Streptomyces hygroscopicus* bar-gene, which encodes phosphinothricin acetyltransferase (an enzyme which inactivates the herbicide phosphinothricin), into embryogenic cells of a maize suspension culture by microprojectile bombardment (Plant Cell, 1990, 2, 603-618). The introduction of genetic material into aleurone protoplasts of other monocot crops such as wheat and barley has been reported (Plant Mol. Biol., 1989, 13, 21-30). Wheat plants have been regenerated from embryogenic suspension culture by selecting only the aged compact and nodular embryogenic callus tissues for the establishment of the embryogenic suspension cultures (Bio/Technol., 1990, 8, 429-434). The combination with transformation systems for these crops enables the application of the present invention to monocots.

Monocotyledonous plants, including commercially important crops such as rice and corn are also amenable to DNA transfer by *Agrobacterium* strains (vide WO 94/00977; EP 0 159 418 B1; Plant. Physiol., 1991, 95, 426-434; The Plant J., 1994, 6, 271-282).

Following DNA transfer and regeneration, putatively transformed plants may be evaluated, for instance using Southern analysis, for the presence of the DNA coding for a regulatory gene, copy number and/or genomic organization. After the initial analysis, transformed plants showing the desired copy number and expression level of the newly introduced DNA according to the invention may be tested for increased expression of health-promoting substances.

Following such evaluations, the transformed plants may be grown directly, but usually they may be used as parental lines in the breeding of new (inbred) varieties or in the creation of hybrids and the like.

These plants, including plant varieties, with increased production and content of health-promoting substances may be grown in the field, in the greenhouse, or at home or elsewhere. Plants or edible parts thereof are preferably used for animal feed or human consumption, or they may be processed for food, feed or other purposes in any form of agriculture or industry. Agriculture shall mean to include horticulture, arboriculture, flower culture, and the like. Industries which may benefit from plant material according to the invention include but are not limited to the pharmaceutical industry, sugar manufacturing industry, feed and food industry, and the like.

Plants for the purpose of this invention shall mean multicellular organisms capable of photosynthesis, and subject to some form of pathogen induced disease. They shall at least include angiosperms as well as gymnosperms, monocotyledonous as well as dicotyledonous plants.

An intrinsic effect of the overexpression of an RKS receptor in a plant is next to the above described enhanced capacity to form health-promoting substances, also the fact that the plant is primed, i.e. that it will more readily be able to effects its autonomous antipathogenic measures after a pathogen attack. Thus, RKS overexpressing plants are more vigourous than their non-transgenic counterparts.

### EXAMPLES

### Example 1. Metabolic changes in RKS4 transgenic plants.

Metabolite analysis was performed essentially as described by Jahangir et al. (Food Chem. (2008) 107(1): 362-368) using ¹H-NMR on total extracts from lyophilised rosette leaves of plants overexpressing the full-length or a modified form of the *RKS4* gene. Arabidopsis plants were grown on soil in a growth chamber at 21°C and 65% relative humidity with a 16h photoperiod (100 µmol.m⁻².s⁻¹). The rosette of 1 month-old Arabidopsis plants was harvested for 5 individual plants of each line. Each rosette was lyophilised and further analysed individually as an independent sample. An example of spectra for 2 samples is shown in Figure 1. After normalisation of signal intensities of the NMR spectra, differences between samples (individual rosettes) were identified and categorised using Multivariate Data Analysis (Principal Component Analysis and Partial Least Square-Discriminant Analysis). Differential metabolites between the transgenic lines and the wild-type are shown in the table hereafter (Table 1).

**Table 1. Metabolites with abundance changes in RKS4 plants Higher abundance is indicated by a'+', whereas lower abundance is indicated by a '-'.**

| **Metabolite** | **Change in transgenic plants vs. wild-type** |
|---|---|
| Alanine | + |
| Betaine analogue | - |
| Choline | + |
| Formic acid | - |
| Fumaric acid | + |
| GABA | + |
| Gallic acid | - |
| Glucose | + |
| Sinigrin (a glucosinolate) | + |
| Glutamic acid | + |
| Glutamine | + |
| Kaempferol-3,7-*O*-dirhamnoside (kaempferitrin) | + |
| Kaempferol-3-*O*-glucose-7-*O*-rhamnoside | + |
| Quercetin | + |
| Proline | + |
| two Sinapic acid analogues | + |
| Sucrose | + |
| Threonine | + |

### Example 2. The GABA shunt is modulated in RKS4 transgenic plants.

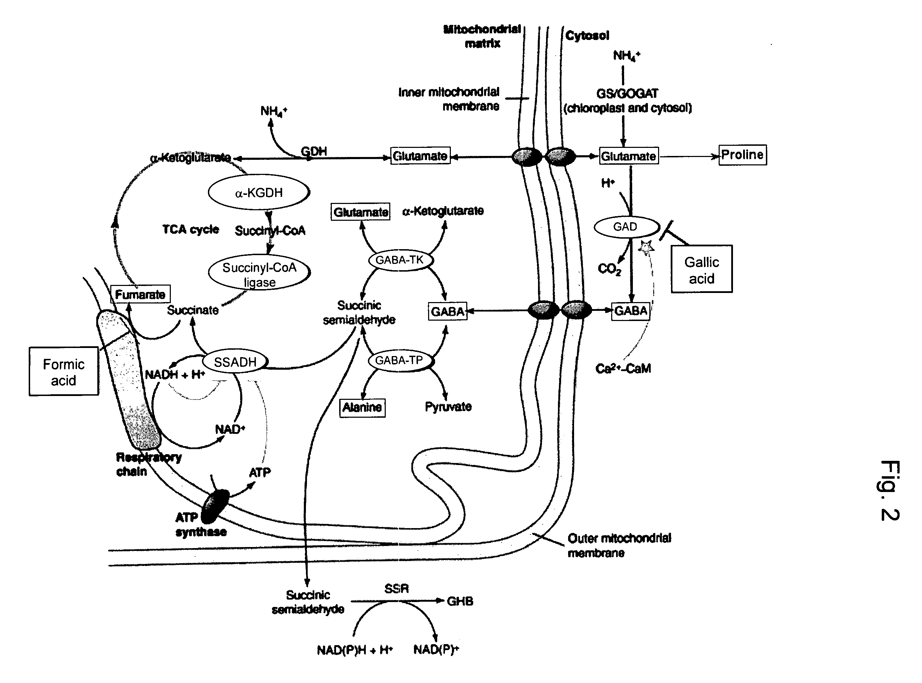

Changes in metabolites as shown in Table 1 are in line with a modulation of the GABA shunt metabolic pathway (Figure 2). The latter is known to be rapidly activated in plants by several stresses that cause the production of reactive oxygen species (ROS, TIPS (2004) 9(3): 110-115). The level of GABA, for which there is increasing evidence for a role in stress tolerance (Crit. Rev. Plant Sci. (2000) 19: 479-509), is increased in the RKS4 plants together with that of e.g. glutamate, fumarate, alanine and proline. Increase in these compounds is concomitant with a decrease in gallic acid that inhibits the enzyme glutamate decarboxylase (GAD) converting glutamate into GABA and with a decrease in formic acid that inhibits the conversion of succinate into fumarate within the TCA cycle (see Table 1). Interestingly GAD is actively induced in *E. coli* when exposed to stress and is hypothesized to contribute to cytosolic pH regulation through GABA production (TIPS (2004) 9(3): 110-115). In turn GABA is also a precursor of alanine, which level is higher in RKS4 plants and glutamate is a precursor of proline, which level is also elevated in the same plants (Table 1). Increased proline levels are also commonly associated with osmotic and salt stress (Plant Phys. (1998) 117(1): 263-271 and Physiol. Plant. (2004) 120(3): 442-450, respectively). These results are in lines with a role of RKS4 in stress tolerance in plants (Patent application no. EP 0710962) but also with the therapeutic use of such plants for animals and humans, based on the roles of GABA as anxiolytic, anticonvulsant and pituitary stimulant.

### Example 3. Metabolic changes in RKS4 plants after elicitation (e.g. bacterial infection)

A transcriptome analysis of RKS4 transgenic plants was performed after challenge inoculation with *Pseudomonas syringae* pv. tomato DC3000 (AvrPst).

Five week-old Arabidopsis plants (15 per line) overexpressing the full-length or a modified form of the *RKS4* gene were inoculated with the bacterium and flash frozen at 3 time points (6, 24 and 48 hours post inoculation - 5 plants per line) for RNA isolation. Expression analysis was performed by hybridisation to Agilent 4x 44K Arabidopsis 3 Oligonucleotide microarrays and data was analysed using the Genespring GX software. Lists of significant differentially expressed genes as compared to the wild-type (more than 2-fold changes) were established per time point as well as in a time-course manner. A battery of defense-related genes was found to be strongly influenced in several lines after *Pseudomonas syringae* infection. An example of genes having the same behaviour at all 3 time points is shown hereafter (Table 2). Based on the annotations of these target genes one can already see that defense-related genes are over-represented.

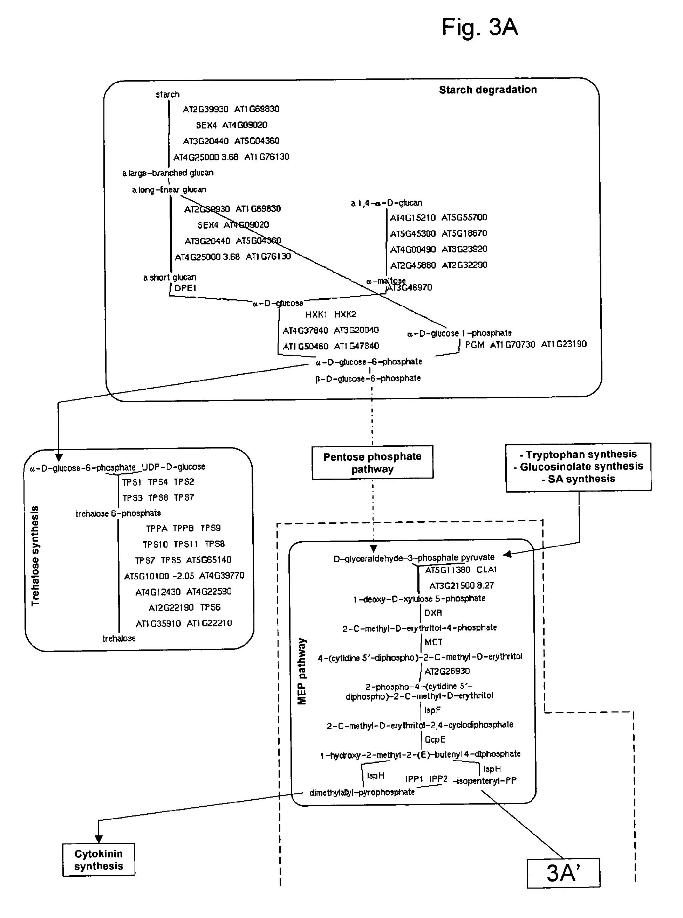

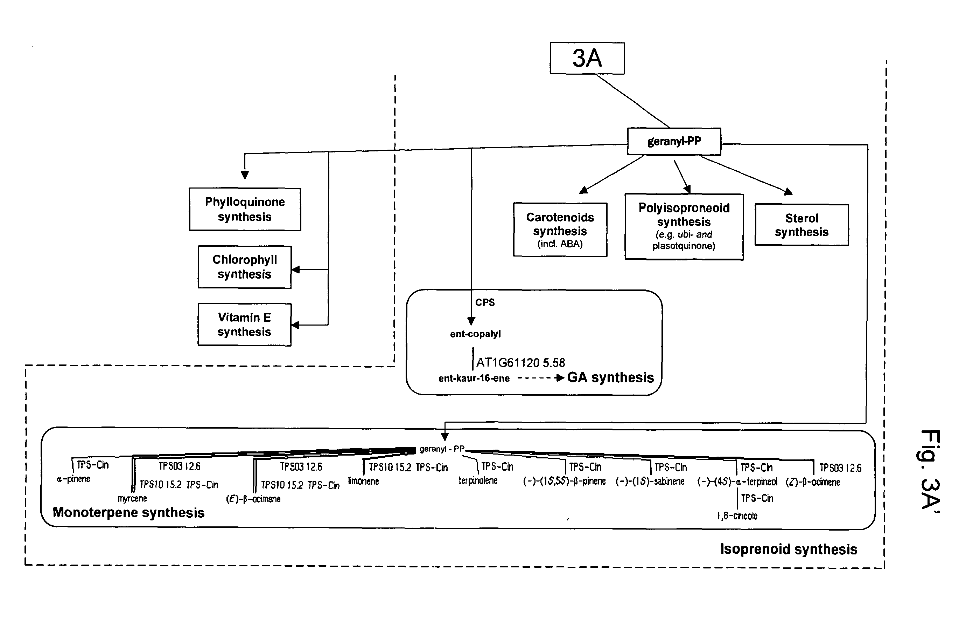

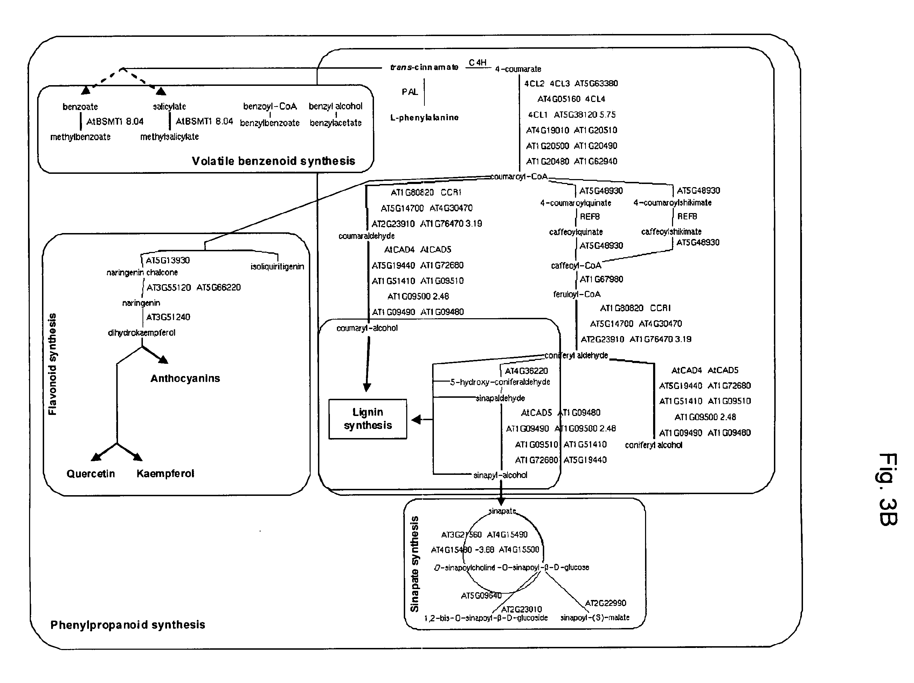

To gain further insight in the putative function of the identified genes the results in Table 2 were subjected to the 'Pathway Tools Omics Viewer' at The Arabidopsis Information Resource (www.arabidopsis.org). Genes for which a link has already been established with a metabolic pathway are highlighted on the corresponding reaction with a colour related to the expression level. The individual pathways thereby identified were copied from the tool and are represented in Figure 3. Links could be made in a number of cases between several pathways leading to the definition of two main biosynthesis pathways: isoprenoids and phenylpropanoids (figures 3A and 3B, respectively). Both classes of metabolites are associated with plant defense and in view of the links established by our analysis are at the basis of the primed state induced by the (modified) RKS4 receptor, possibly in combination with yet to be identified pathways. In addition to their role in plant defence these classes of metabolites are also associated with human health (Curr. Topics Nutr. Res. (2004) 2(1): 47-65).

For example the isoprenoid synthesis pathway was found as a central point and more specifically the methylerythritol-4-phosphate (MEP) pathway also known as non-melavonate (MVA) pathway. Noteworthy is the up-regulation of the gene coding for DXPS1 (1-deoxy-D-xylulose-5-phosphate synthase, At3g21500, see also Table 3), which catalyses the rate-limiting step in plastidic isoprenoid synthesis (J. Biol. Chem. (2001) 276(25):22901-22909). This enzyme is also the target of choice for metabolic engineering of this pathway (Plant Biotechnol. J. (2005) 3(1):17-27; Nature Chem. Biol. (2007) 3(7): 387-395). An increase in its expression in the RKS4 plants is therefore in agreement with an increase in precursors of the MEP pathway and consequently in isoprenoid synthesis. The downstream effect, i.e. the actual isoprenoids of which the level is influenced is at this moment unknown and one can only conclude based on the identified genes that at least terpene production as well as giberellin production are increased.

**Table 2. Differentially regulated genes after AvrPst infection in RKS4 line GT19-A14).**

| Normalized fold expression changes are indicated for each time point after | | | |
|---|---|---|---|
| infection with the bacterium (HPI: Hours Post-Infection). | | | |
| **Up-regulated genes after AvrPst infection in RKS4** | | | |
| | | **AvrPst** | |
| **line GT19-A14** | | | |
| **Annotation** | **6HPI** | **24HPI** | **48HPI** |
| 4-coumarate--CoA ligase/ 4-coumaroyl-CoA synthase | | | |
| family protein; phenylpropanoid pathway - defense- | 3.85 | 2.34 | 5.75 |
| related. | | | |
| adenylosuccinate lyase, putative / adenylosuccinase, | 2.56 | 2.06 | 7.69 |
| putative (TCA cycle, putative). | | | |
| alcohol oxidase-related (TCA cycle, putative) | 2.50 | 2.22 | 4.10 |
| AMY1 (ALPHA-AMYLASE-LIKE); alpha-amylase - | 2.22 | 3.31 | 3.68 |
| Response to GA. | | | |
| ANAC019 (Arabidopsis NAC domain containing | 2.42 | 2.83 | 2.80 |
| protein 19); transcription factor - Response to stress. | | | |
| AP2 domain-containing transcription factor, putative | 6.81 | 8.12 | 2.36 |
| ARABIDILLO-2 / F-box family protein - proteasome. | | | |
| | 2.73 | 2.67 | 3.11 |
| (Patent stress tolerance) | | | |
| ATNRT2.6 (Arabidopsis thaliana high affinity | | | |
| | 2.06 | 2.20 | 4.39 |
| nitrate transporter 2.6); nitrate transporter | | | |
| ATPP2-B6 (Phloem protein 2-B6); similar to Cyclin- | | | |
| | 2.89 | 3.27 | 3.91 |
| like F-box - proteasome. | | | |
| ATTPS03 (Arabidopsis thaliana terpene synthase 03) | | | |
| | 4.78 | 3.72 | 12.64 |
| - Response to wounding. | | | |
| BGLU45; hydrolase, hydrolyzing O-glycosyl | | | |
| 2.31 2.30 3.72 | | | |
| compounds | | | |
| *bHLH family protein, TF similar to NAI1* | *2.32* | *3.27* | *6.75* |
| *bHLH family protein, TF similar to NAI1* | *2.13* | *5.12* | *8.02* |
| BT2 (BTB and TAZ domain protein 2); an essential | | | |
| component of the TAC1-mediated telomerase | 5.42 | 2.52 | 3.18 |
| activation pathway | | | |
| CBF1 (C-REPEAT/DRE BINDING FACTOR 1); | | | |
| | 2.01 | 3.97 | 2.96 |
| transcription factor - Response to stress. | | | |
| cinnamoyl-CoA reductase - lignin biosynthesis. | 2.21 | 2.08 | 3.19 |
| cinnamyl-alcohol dehydrogenase family / CAD | | | |
| | 2.49 | 2.23 | 2.48 |
| family; phenylpropanoid pathway - defense-related: | | | |
| copine-related | 2.12 | 2.33 | 3.51 |
| CYP94B3 (cytochrome P450, family 94, subfamily B, | | | |
| | 2.04 | 3.95 | 7.35 |
| polypeptide 3); oxygen binding | | | |
| Cytochrome b, putative; electron transport. | 4.10 | 3.37 | 4.10 |
| DXPS1; 1-deoxy-D-xylulose-5-phosphate synthase; | | | |
| | 5.10 | 3.91 | 8.27 |
| terpenoid biosynthesis. | | | |
| ELI3-2 (Elicitor-Activated Gene 3); Cinnamyl- | | | |
| | 3.50 | 2.60 | 2.35 |
| Alcohol Dehydrogenase - Response to bacteria. | | | |
| esterase/lipase/thioesterase family protein | 2.12 | 2.06 | 3.06 |
| extracellular dermal glycoprotein-related / EDGP- | 3.41 | 2.44 | 6.22 |
| related - Response to (a)biotic stresses. | | | |
| extracellular dermal glycoprotein-related / EDGP- | 3.21 | 2.10 | 4.20 |
| related - Response to (a)biotic stresses. | | | |
| FLR1 (FLOR1); LRR protein - Interaction with VSP1 | | | |
| | 3.11 | 2.48 | 4.82 |
| and AGAMOUS. | | | |
| GCN5-related N-acetyltransferase (GNAT) family | | | |
| | 3.21 | 4.26 | 4.52 |
| protein - Histone acetylation. | | | |
| Glutamine amidotransferase class-I, putative - | 11.87 | 20.58 | 28.29 |
| defense-related protein, putative. | | | |
| Glutamine amidotransferase class-I, putative - | 10.4 | 9.33 | 9.23 |
| defense-related protein, putative. | | | |
| Glycine-rich protein; electron transport. | 4.12 | 3.19 | 2.96 |
| hydrolase, alpha/beta fold family protein, similar to | | | |
| ACL (Acetone-Cyanohydrin Lyase), similar to | 2.55 | 3.29 | 4.16 |
| ethylene-induced esterase. | | | |
| ILL6 (IAA-leucine resistant (ILR)-like gene 6); | 2.03 | 2.09 | 3.14 |
| metallopeptidase - Clubroot disesase in B. napus. | | | |
| jacalin lectin family protein, similar to MBP1 | 2.57 | 2.22 | 3.74 |
| (Myrosinase-Binding Protein 1) - response to stress. | | | |
| jacalin lectin family protein; Myrosinase-Binding | 2.20 | 2.00 | 3.95 |
| Protein - response to stress. | | | |
| *JAZ10* = *TIFY9; similar to ZIM- Response to JA.* | *2.24* | *2.74* | *4.35* |
| JAZ7 = TIFY5b; similar to ZIM - Response to JA. | 2.39 | 2.61 | 3.81 |
| Kelch repeat-containing F-box family protein; similar | 2.66 | 2.30 | 2.66 |
| to autophagy 4a (APG4a) - proteasome. | | | |
| KID (kinase-inducible domain )-containing protein - | 2.85 | 2.05 | 2.95 |
| stress-induced. | | | |
| latex-abundant protein, putative (AMC4) / caspase | 2.39 | 3.18 | 6.95 |
| family protein - PCD (response to Botrytis). | | | |
| Lyase/ magnesium ion binding | 8.01 | 5.56 | 10.09 |
| MAF5 (MADS AFFECTING FLOWERING 5); | 2.31 | 2.48 | 2.32 |
| transcription factor - Vernalization. | | | |
| MAPKKK19 (Mitogen-activated protein kinase | 2.21 | 3.99 | 2.75 |
| kinase kinase 19) - Response to pathogens & JA. | | | |
| MDAR3 (MONODEHYDROASCORBATE | 4.13 | 2.00 | 4.05 |
| REDUCTASE) - Response to JA. | | | |
| methionine sulfoxide reductase domain-containing | | | |
| | 2.76 | 4.79 | 3.05 |
| protein / SeIR domain-containing protein - PCD. | | | |
| MYB113 (myb domain protein 113) - Response to JA. | 2.98 | 2.14 | 5.02 |
| Myrosinase-associated protein, putative; | 4.47 | 2.07 | 4.48 |
| glucosinolate metabolism - defense-related, putative. | | | |
| N-myristoylation protein, putative; signal | | | |
| | 3.89 | 2.53 | 4.62 |
| transduction. | | | |
| nucleoside phosphatase family protein / GDA1/CD39 | 3.68 | 2.88 | 15.01 |
| family protein | | | |
| nucleoside phosphatase family protein / GDA1/CD39 | 3.28 | 3.61 | 12.39 |
| family protein | | | |
| nucleoside phosphatase family protein / GDA1/CD39 | 2.67 | 2.79 | 5.86 |
| family protein | | | |
| O-methyltransferase family 2 protein | 2.55 | 3.05 | 3.43 |
| Oxidoreductase | 5.48 | 6.70 | 7.96 |
| oxidoreductase, 2OG-Fe(II) oxygenase family protein | 2.94 | 2.51 | 2.27 |
| oxidoreductase, 2OG-Fe(II) oxygenase family protein | 2.91 | 2.61 | 3.25 |
| - Anthocyianidin synthase (ANS). | | | |
| palmitoyl protein thioesterase family protein | 2.87 | 2.30 | 5.07 |
| Phosphorylase family protein; similar to vegetative | 4.60 | 3.45 | 10.41 |
| storage protein. | | | |
| plastocyanin-like domain-containing protein | 2.43 | 2.58 | 3.18 |
| PRN (PIRIN); calmodulin binding - ABA response, | 2.71 | 2.28 | 0.49 |
| putative. | | | |
| protease inhibitor/seed storage/lipid transfer protein | 3.46 | 3.96 | 2.47 |
| (LTP) family protein | | | |
| RD21 (RESPONSIVE TO DEHYDRATION 21); | 2.50 | 2.44 | 5.36 |
| cysteine-type peptidase - Response to stress. | | | |
| RGL3 (RGA-LIKE 3);DELLA transcription factor - | 2.48 | 2.05 | 2.98 |
| Response to GA. | | | |
| S-adenosyl-L-methionine:carboxyl methyltransferase | | | |
| family protein BSMT1 - Methylation of benzoic acid | 3.66 | 3.52 | 8.04 |
| and salicylic acid - Defense response. | | | |
| senescence-associated protein-related | 2.61 | 2.56 | 3.18 |
| Similar to 2-oxoglutarate-dependent dioxygenase, | 3.00 | 3.28 | 4.68 |
| putative - Flavonol metabolism, putative. | | | |
| Similar to Asparagine Synthetase (PTHR11772); | | | |
| contains domain N-terminal nucleophile | 2.79 | 2.48 | 11.14 |
| aminohydrolases. | | | |
| Similar to CYP704A2 (cytochrome P450, family 704, | 3.98 | 3.99 | 3.17 |
| subfamily A, polypeptide 2); oxygen binding. | | | |
| similar to DRM1; dormancy/auxin associated family | 2.27 | 2.27 | 2.15 |
| protein | | | |
| similar to FRK1 (FLG22-Induced Receptor-Like | 2.55 | 2.00 | 2.26 |
| Kinase 1) - Early defense signaling. | | | |
| Subunit of mitochondrial NAD(P)H dehydrogenase | | | |
| (trans-spliced from three precursors, NAD1A, | 5.73 | 4.81 | 4.92 |
| NAD1B & NAD1C). | | | |
| terpene synthase/cyclase family protein; similar to | | | |
| GA2 (GA REQUIRING 2), ent-kaurene | 2.78 | 2.79 | 5.58 |
| synthase;similar to linalool synthase. | | | |
| TPS10 (TERPENE SYNTHASE 10); myrcene/(E)- | 5.91 | 5.05 | 15.15 |
| beta-ocimene synthase - Response to JA. | | | |
| tryptophan synthase, beta subunit, putative | 3.50 | 3.11 | 11.20 |
| UDP-glucoronosyl/UDP-glucosyl transferase family | 2.69 | 2.26 | 2.11 |
| protein | | | |
| UDP-glycosyltransferase/ transferase, transferring | 2.38 | 2.08 | 2.56 |
| glycosyl groups | | | |
| unknown protein | 3.55 | 2.23 | 2.47 |
| unknown protein | 2.75 | 2.06 | 3.30 |
| unknown protein | 2.73 | 2.24 | 17.62 |
| unknown protein, contains DUF506 | 3.23 | 2.10 | 3.42 |
| VSP1 (VEGETATIVE STORAGE PROTEIN 1); acid | 4.23 | 3.61 | 9.55 |
| phosphatase - Response to JA. | | | |
| VSP2 (VEGETATIVE STORAGE PROTEIN 2); acid | 3.38 | 4.34 | 16.34 |
| phosphatase - Response to JA. | | | |
| Wall-associated receptor kinase-like 18 precursor - | | | |
| | 2.04 | 2.36 | 6.60 |
| defense-related. | | | |
| WRKY62; transcription factor - NPR1 signaling | 3.28 | 2.21 | 4.50 |
| (cross-talk SA/JA). | | | |
| (continuing) | | | |

| **Down-regulated genes after AvrPst infection in** | **AvrPst** | | |
|---|---|---|---|
| **RKS4 line GT19-A14** | | | |
| **Annotation** | **6HPI** | **24HPI** | **48HPI** |
| Contains domain Cupin, RmlC-type, synthesis of L- | -8.26 | -3.40 | -8.62 |
| rhamnose, required for bacterial virulence. | | | |
| unknown protein | -8.06 | -8.06 | -17.92 |
| protein kinase family protein, U-box domain - | -5.21 | -3.72 | -6.67 |
| proteasome. | | | |
| germin-like protein, putative | -5.00 | -0.20 | -3.07 |
| no_match | -4.98 | -3.73 | -6.71 |
| ELP1 (EDM2(enhanced downy mildew 2)-LIKE | | | |
| PROTEIN1); zinc ion binding TF Response to H. | -4.95 | -2.21 | -4.50 |
| parasitica. | | | |
| trehalose-6-phosphate phosphatase, putative | -4.37 | -3.40 | -2.05 |
| bHLH family protein | -4.26 | -5.32 | -2.26 |
| no_match | -4.00 | -0.19 | -4.10 |
| acyl-CoA thioesterase family protein | -3.85 | -2.11 | -3.03 |
| zinc ion binding | -3.69 | -3.56 | -2.42 |
| monooxygenase family protein, similar to CTF2B - | -3.56 | -2.99 | -3.36 |
| ABA synthesis, putative. | | | |
| UBC8 (UBIQUITIN CONJUGATING ENZYME 8); | -3.55 | -2.39 | -2.23 |
| ubiquitin-protein ligase - proteasome. | | | |
| Multi Antimicrobial Extrusion (MATE) efflux family | -3.37 | -2.25 | -2.66 |
| protein | | | |
| oxidoreductase, 2OG-Fe(II) oxygenase family protein | -3.30 | -4.20 | -4.65 |
| protein kinase (ADK1), identical to dual specificity | -3.13 | -3.27 | -2.06 |
| kinase 1 (ADK1) | | | |
| ankyrin protein kinase, putative APK1; | -3.09 | -2.24 | -2.96 |
| similar to TRFL5, HD TF (Telomere-binding). | -2.95 | -3.52 | -5.56 |
| ELIP2 (EARLY LIGHT-INDUCIBLE PROTEIN 2); | -2.92 | -2.92 | -5.32 |
| chlorophyll binding | | | |
| amino acid transporter family protein | -2.89 | -3.22 | -3.10 |
| no_match | -2.83 | -2.29 | -2.01 |
| contains DUF300 | -2.82 | -2.10 | -0.45 |
| unknown protein | -2.82 | -4.52 | -2.99 |
| defensin-like (DEFL) family protein, contains | -2.81 | -2.31 | -6.99 |
| domain CYS_RICH (PS50311) | | | |
| unknown protein | -2.68 | -3.16 | -2.16 |
| contains DUF1298 | -2.67 | -2.37 | -3.50 |
| unknown protein | -2.60 | -2.37 | -2.24 |
| UGT84A1; UDP-glycosyltransferase/ sinapate 1- | | | |
| | -2.51 | -2.17 | -3.68 |
| glucosyltransferase/ transferase. | | | |
| ORG3 (OBP3-responsive gene 3); bHLH TF - SA- | | | |
| | -2.44 | -2.77 | -5.92 |
| induced & JA-inhibited. | | | |
| unknown protein | -2.36 | -2.43 | -4.02 |
| Cytochrome c biogenesis orf382 | -2.27 | -2.31 | -2.40 |
| unknown protein | -2.21 | -2.62 | -3.06 |
| ORG2 (OBP3-responsive gene 2); bHLH TF - SA- | | | |
| | -2.21 | -2.07 | -5.81 |
| induced & JA-inhibited. | | | |
| AtLon3, Lon protease, putative - Organelle | | | |
| | -2.18 | -5.46 | -2.21 |
| proteolysis. | | | |
| similar to FRL2 (FRIGIDA LIKE 2); similar to ABI3- | | | |
| | -2.16 | -2.49 | -2.05 |
| interacting protein 2 - Vernalization. | | | |
| no_match | -2.13 | -2.95 | -2.91 |
| SNOR108, Encodes a C/D box snoRNA - site-specific | | | |
| | -2.10 | -2.79 | -2.37 |
| RNA methylation. | | | |
| UGT72C1 (LTDP-glucosyl transferase 72C1); UDP- | | | |
| | -2.08 | -3.83 | -11.07 |
| glycosyltransferase/ transferase - Pst-induced. | | | |
| (continued) | | | |

## Claims

1. Method to enhance the production of plant-specific compounds by increasing expression of a regulatory gene product.

2. Method according to claim 1, wherein said compounds have health-promoting properties.

3. Method according to claim 1 or 2, wherein said regulatory gene product is a receptor kinase, more preferably a RKS receptor or a mutein thereof or a chimeric RKS receptor.

4. Method according to any of claims 1 to 3 comprising providing a plant with a nucleotide sequence encoding said RKS receptor.

5. Method according to claim 3 or 4, wherein said RKS gene produces a truncated RKS protein.

6. Method according to any of claims 3-5, wherein said RKS gene is chosen from the group consisting of RKS subgroup I (RKS1, RKS4, RKS5, RKS7, RKS11, and RKS14), more specifically RKS4 and truncated RKS4 and chimeric receptors comprising the extracellular domain of RKS4.

7. Method according to any of the previous claims wherein the health-promoting capacity of a plant or plant product is enhanced by increasing the production in said plant of health-promoting substances selected from the group of polyphenols, glucosinolates, isoprenoids, and GABA and analogs.

8. Method according to claim 7, where the substances are chosen from the group consisting of quercetin, kaempferol, sinapic acid, gallic acid, GABA and sinigrin.

9. Method to produce a health-promoting substance in a plant by introducing into said plant a gene coding for a regulatory gene product, preferable a receptor kinase, more preferable an RKS gene.

10. Use of an RKS gene for the production of health-promoting substances in a plant.

11. Plant with an enhanced health-promoting capacity produced by the method according to any of claims 1-8.
